# EUROPEAN PATENT APPLICATION

(11) **EP 1 669 368 A1**
(43) Date of publication of application: **14.06.2006**
(21) Application number: 04292964.6
(22) Date of filing: 13.12.2004
(51) Int. Cl.: C07K 14/15, A61K 38/16, G01N 33/50, A61L 2/00, A61P 31/14

(54) **Use of peptides from the E2 protein of HCV for treating HCV infection**

(71) Applicant: Genoscience, 13006 Marseille (FR)
(72) Inventor: Sabatier, Jean-Marc, 13790 Rousset (FR); Halfon, Philippe, 13018 Marseille (FR)
(74) Representative: Tezier Herman, Béatrice

(57) **Abstract**

The present invention relates to peptides or derivatives thereof, pharmaceutical compositions comprising the same and uses thereof to treat or prevent a viral infection, in particular a retroviral infection. The invention specifically discloses novel chemical compounds as active ingredients to treat or prevent such infection, in particular HCV infection, by inhibiting the fusion of said virus with target cells.

## Description

### FIELD OF THE INVENTION

The present invention relates to peptides or derivatives thereof, pharmaceutical compositions comprising the same and uses thereof to treat a viral infection, in particular a retrovirus infection, such as Hepatitis C Virus (HCV) infection. The invention specifically discloses novel chemical compounds as active ingredients to treat such infection.

### BACKGROUND OF THE INVENTION

Hepatitis C virus (HCV) is a major cause of chronic hepatitis worldwide. Most HCV-infected individuals develop chronic hepatitis that may progress to liver cirrhosis and hepatocellular carcinoma.
HCV is a member of the flavivirus family of retroviruses. Retroviruses are small enveloped viruses that contain a diploid, single-stranded RNA genome, and replicate via a DNA intermediate produced by a virally-encoded reverse transcriptase, an RNA-dependent DNA polymerase. Other retroviruses include, for example, HIV (human immunodeficiency virus), SIV, FIV, BIV, VISNA, CAEV, EIAV, oncogenic viruses, such as human T-cell leukaemia viruses (HTLV-I,-II,-III), and feline leukemia virus.
HCV is a small, enveloped, plus-strand RNA virus belonging to the family flaviviridae and genus hepacivirus. The HCV RNA genome is 9600 nucleotides in length and encodes a single polyprotein that is post-translationally cleaved into up to 10 polypeptides including three structural proteins (core, E1 and E2), located at the N-terminus, and five nonstructural proteins. The transmembrane domains of E1 and E2 are responsible for both heterodimerization and retention of the glycoproteins in a high mannose EndoH sensitive glycoform in the endoplasmic reticulum (ER). By analogy to other flaviviruses, it is assumed that HCV capsids bud from the cytoplasm into the ER and that enveloped particles follow the secretion pathway through the Golgi. However, attempts to produce secreted HCV particles *in vitro* have not been successful so far, and it is not known if E1 and E2 on mature infectious virions possess a high-mannose, complex or mixed glycosylation.
Since the genomic sequence of HCV was determined, significant progress has been made towards understanding the functions of the HCV-encoded proteins, despite the lack of an efficient *in vitro* replication system or convenient small-animal models. The identity of the receptor for HCV remains elusive. Several receptors have been proposed that could play a role in HCV entry into hepatocytes. Low-density lipoprotein receptors, CD81, SR-BI and GAGs may act as receptors for HCV, either sequentially or by different viral quasispecies. The low density lipoprotein (LDL) receptor (LDLR) has been shown to mediate HCV internalization by binding to virus associated LDL particles. A second putative HCV receptor, the tetraspanin CD81, has been identified as a high affinity binding receptor for soluble recombinant E2 from HCV genotype 1a. CD81 and LDLR are expressed in most cell types and thus likely do not account for the hepatic tropism of the virus. Recent work using pseudotypic VSV bearing E1 or E2 chimeric molecules showed that entry of the E1 pseudotype can be inhibited by recombinant LDLR, whereas the E2 pseudotype is more sensitive to inhibition by recombinant CD81 or heparin. These results suggest that E1 and E2 may be responsible for interactions with different cellular molecules. It is also conceivable that additional, yet unidentified, cellular proteins are involved in viral binding and entry (i.e. : viral fusion). Intriguingly, the reports of HCV-RNA associated with PBMC (peripheral blodd mononuclear cells) suggest that HCV infection may not be restricted to hepatocytes. Thus, separate reservoirs of virus may exist, and HCV may use different receptors to access these different cell types. Scarselli et al (EMBO J. 2002 Oct 1;21(19):5017-25) discovered that the HCV envelope glycoprotein E2 binds to human hepatoma cell lines independently of the previously proposed HCV receptor CD81. They have identified the receptor responsible for E2 binding to human hepatic cells as the human scavenger receptor class B type I (SR-BI). E2-SR-BI interaction is very selective, since neither mouse SR-BI nor the closely related human scavenger receptor CD36 was able to bind E2. Finally, E2 recognition by SR-BI was competed out in an isolate-specific manner both on the hepatoma cell line and on the human SR-BI-transfected cell line by an anti-HVR1 monoclonal antibody.

Currently, there are more than 400 million people infected with HCV worldwide *(J. Virol.* 2000; 74: 5747-6229). Although considerable effort is being put into the successful design of effective therapeutics, currently a vaccine for prevention of infection is not available and treatment options for chronic HCV infection are limited. The only available therapy is interferon-α in combination with ribavirin. This treatment is expensive and shows only moderate response rates and presents risk of significant side effects. Consequently, the development of diagnostic tools, improved therapies and both therapeutic and prophylactic vaccines against HCV are high-priority goals.
It is therefore an object of the present invention to provide chemical compounds useful in the treatment or prevention or amelioration of one or more symptoms of hepatitis C virus infection and related infections.

### SUMMARY OF THE INVENTION

The invention provides a class of chemical compounds useful as therapeutic agents for preventing or treating HCV infection or any infection due to any other retrovirus. It is a further object to provide compositions containing at least one of these compounds useful for the treatment or prevention or amelioration of diseases related or due to an infection by a virus, in particular a retrovirus, such as HCV or feline leukemia virus. A particular embodiment is to provide compositions containing at least one of these compounds useful for the treatment or prevention or amelioration of one or more symptoms of HCV infection. Another object is to provide methods for production of other antiviral agents having activity against a retrovirus, such as HCV (Hepatitis C Virus), more particularly that can inhibit fusion of virus (e.g, HCV) with target cells, by using said compounds. It is a further object to provide methods for preparing the compounds. It is a further object to provide the use of these compounds for the preparation of compositions useful in the treatment, the prevention, or the amelioration of diseases related or due to an infection by a virus and their use for production of other antiviral agents having activity against a virus (e.g., HCV). These compositions are in particular useful in the treatment, the prevention, or the amelioration of one or more of the symptoms of HCV. In particular, compounds of the invention blocks HCV's ability to infect healthy hepatocytes or any other target cells.
When used with other anti-HCV agents (interferon-α, ribavirine), compounds of the invention could reduce the amount of HCV in the blood.
A further object of the invention is to provide methods for inhibiting fusion of retrovirus, in particular HCV, using the compounds of the invention.
Another object herein is to provide *in vitro* model useful for investigating retrovirus (e.g., HCV) infection and particularly the process of fusion or transmission of retrovirus (e.g., HCV).

### DETAILED DESCRIPTION OF THE INVENTION

The present invention discloses polypeptides comprising the amino acid sequence (I) : YPYRLWHYPXTVNFTIFKVRMYVGGMEHR (I) (SEQ ID No. 1), wherein X is C or an amino acid derivative thereof devoid of a SH group, such as α-aminobutyrate, or an equivalent amino acid residue; a fragment thereof; a variant thereof or any other polypeptide-mimicking molecule.

In a particular embodiment, polypeptides of the invention comprise the amino acid sequence (II) : GPWLTPRXIVDYPYRLWHYPXTVNFTIFKVRMYVGGMEHR (SEQ ID No. 2), wherein X is as defined above.

The polypeptides, which chemical structures are detailed below, are potent inhibitors of retrovirus infection, in particular of HCV infection, being active in the micromolar concentration range in *in vitro* assays, as shown below. These polypeptides are able to inhibit cell-to-cell transmission of HCV with an undefined mechanism, as well as virus-to-cell infection (in particular by inhibition of HCV fusion with target cells, such as hepatocytes) *in vitro.* As shown below, the polypeptides are also able to inhibit cell-to-cell transmission of other retrovirus, such as feline leukemia virus, as well as virus-to-cell infection *in vitro.*
The molecules are neither toxic at bioactive peptide concentrations (up to 50 micromolar), nor lethal by intracerebroventricular inoculation in C57/BL6 mice (up to 200 micrograms per mouse).
In view of that properties, the polypeptides of the invention are particularly useful as active ingredients to prevent or treat a disease associated with an infection by a virus, in particular a retrovirus, such as HCV and feline leukemia virus.

The term "polypeptide" refers to an isolated polymer of amino acids without regard to the length of the polymer; thus, peptides, oligopeptides, and proteins are included within the definition of polypeptide. This term also does not specify or exclude post-expression modifications of polypeptides, for example, polypeptides which include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art, both naturally occurring and non-naturally occurring.
The polypeptides according to the invention are preferably from three (e.g. NFT) amino acid residues to fifty amino acid residues in length, more preferably from five amino acid residues to twenty, thirty or forty in length, in particular 29 or 40 amino acids, such as the specific amino acid sequence of sequence (I) or (II), in length.

The polypeptides according to the invention include also variants, fragments, analogs and derivatives thereof. Said variants, fragments, analogs and derivatives of the polypeptides described hereinabove have substantially the same functions and properties as the polypeptides defined above, in particular they can be used as active ingredients to prevent or treat a viral infection caused by strains or isolates of HCV or any other pathogenic retroviruses, in particular HIV (human immunodeficiency virus), SIV, FIV, BIV, VISNA, CAEV, EIAV, oncogenic viruses, such as human T-cell leukaemia viruses (HTLV-I,-II,-III), and feline leukemia virus.

The variant may be 1) one in which one or more of the amino acid residues, preferably no more than five amino acid residues, are substituted with a conserved or non-conserved amino acid residue and such substituted amino acid residue may or may not be one encoded by the genetic code, or 2) one in which one or more of the amino acid residues includes a substituent group, or 3) one in which one or more of the amino acid residues is substituted by a chemical compound bound to the other amino acids of the sequence by a or covalent bond(s), or 4) one in which the polypeptide according to the invention is fused, joined or linked to another compound, whereby the compound is capable of providing some (other) useful function as apparent to one skilled in the art in a method according to the present invention ; such compounds are well known in the art to include, but are not limited to, polymers, carbohydrates, phospholipids, and lipid-fatty acid conjugates; such compounds may increase the half-life of the polypeptide (for example, polyethylene glycol), or 4) one in which the additional amino acids are fused to the polypeptide according to the invention, such as a leader or secretory sequence or a sequence which is employed for purification of the polypeptide according to the invention. Such variants are deemed to be within the scope of those skilled in the art.

A polypeptide fragment is a polypeptide having a sequence that is entirely the same as part but not all of the given polypeptide sequence.

In the case of an amino acid substitution in the amino acid sequence of a polypeptide according to the invention, one or several amino acids can be replaced by "equivalent" amino acids, preferably no more than five amino acid residues.
The expression "equivalent" amino acid is used herein to designate any amino acid that may be substituted for one of the amino acids having similar properties, such that one skilled in the art of peptide chemistry would expect the secondary structure and hydropathic nature of the polypeptide to be substantially unchanged. Generally, the following five groups of amino acids represent equivalent changes:(1) A, P, G, E, D, Q, N, S, T; (2) C, S, Y, T; (3) V, I, L, M, A, F; (4) K, R, H; (5) F, Y, W, H.
A specific embodiment of a polypeptide molecule of interest according to the present invention, includes, but is not limited to, a polypeptide molecule which is resistant to proteolysis, a polypeptide in which a or the -CONH- peptide bond is modified and replaced by a (CH2NH) reduced bond, a (NHCO) retro-inverso bond, a (CH2-O) methylene-oxy bond, a (CH2-S) thiomethylene bond, a (CH2CH2) carba bond, a (CO-CH2) cetomethylene bond, a (CHOH-CH2) hydroxyethylene bond), a (N-N) bound, a E-alcene bond or also a -CH=CH- bond. The invention also encompasses a polypeptide, a fragment or a variant thereof in which at least one peptide bond has been modified as described above.
Such fragments may be "free-standing", i.e. not part of or fused to other polypeptides, or they may be comprised within a single larger polypeptide of which they form a part or region.
However, several fragments may be comprised within a single larger polypeptide.

According to a preferred embodiment, the polypeptides according to the invention are those of sequence (I) or (II) wherein X is C, A, S, Y, T or an amino acid derivative thereof devoid of SH group.

Polypeptides according to the invention can present either carboxylic (e.g., -COOH) or preferably carboxyl-aminated (e.g., -CONH₂) C-terminal extremity.

Examples of polypeptides according to the invention include, but are not limited to, the polypeptides of SEQ ID 1 or SEQ ID 2 presenting either carboxylic (e.g., -COOH) or preferably carboxyl-aminated (e.g., -CONH₂) C-terminal extremities. Preferably, in such polypeptides, X represents α-aminobutyrate. More preferably, the polypeptide is selected in the group consisting of :
YPYRLWHYPα-aminobutyrateTVNFTIFKVRMYVGGMEHR, YPYRLWHYPα-aminobutyrateTVNFTIFKVRMYVGGMEHR-NH₂, GPWLTPRα-aminobutyrateIVDYPYRLWHYPα-aminobutyrateTVNFTIFKVRMYVGGMEHR, GPWLTPRα-aminobutyrateIVDYPYRLWHYPα-aminobutyrateTVNFTIFKVRMYVGGMEHR-NH₂, YPα-aminobutyrateTVNFTIFKVRMYVGGMEHR, and YPα-aminobutyrateTVNFTIFKVRMYVGGMEHR-NH₂.

In a particular embodiment, the polypeptides according to the invention can present a lipid moiety, covalently or not covalently attached. The lipid moiety can be any lipid presenting from C3 to C20 carbon atoms, such as valerate or myristate, at N- or C- terminal extremity.
An example of such embodiment is for instance: YPYRLWHYPα-aminobutyrateTVNFTIFKVRMYVGGMEHR-valerate.
According to another embodiment, polypeptides of the invention can comprise at least two or more covalently or non covalently attached polypeptides of amino acid sequence (I) and/or (II). Said sequences can be linear- or branched- attached. The number of repetitions of the sequences (I) and/or (II) can vary from 2 to 8, preferably from 2-4. Said repeated sequences can be interrupted (or linked) by one or several, identical or different, linker(s). Said linkers can be of different natures, including amino acid derivatives, in particular bifunctional amino acid derivatives, including K, ornithine, or any other suitable linker, such as bifunctional hydrocarbonated derivatives, including diaminobutyrate and diaminopropionate, providing said linker does not change substantially the desired effect of the polypeptides according to the present invention.

In a particular embodiment, the present invention provides new branched isolated polypeptides of the following structures : (peptide)₈-K₄-K₂-K, (peptide)₄-K₂-K, (peptide)₂₋K. A polypeptide of such structure is for instance : (YPYRLWHYPα-aminobutyrateTVNFTIFKVRMYVGGMEHR)₈-K₄-K₂-K, (YPYRLWHYPα-aminobutyrateTVNFTIFKVRMYVGGMEHR)₄-K₂-K, or (YPYRLWHYPα-aminobutyrateTVNFTIFKVRMYVGGMEHR)₂-K.

A "polypeptide-mimicking molecule" is a term used to mean a molecule which mimicks the polypeptides as defined above by presenting substantially the same effect, in particular which is able to prevent or treat a viral infection caused by strains or isolates of HCV or any other pathogenic retroviruses. It may include, but is not limited to, a small molecule chemical compound, a natural or synthetic peptide, a mimetic (e.g., peptidomimetic or other mimetic), a polypeptide, an aptamer, a polymer, an oligonucleotide, an antibody or fragment thereof, a polysaccharide, a carbohydrate-containing molecule, an enzyme, an agent, a macromolecule, a metabolite, a combination thereof, and the like. In a preferred embodiment, the compound is a therapeutically deliverable substance; and in a more preferred embodiment, the compound is a therapeutically deliverable substance that can inhibit transmission of virus (e.g. HCV) to a target cell or fusion of virus (e.g. HCV) with a target cell.

The amino acid residues or derivatives within the motif or part of the motif in the polypeptides according to the invention can be L- and/or D-amino acids. In particular, at least one of the amino acid residues of the polypeptides of the invention is a D-amino acid. Polypeptides containing all D-amino acid residues are also included. Example provided is : Y_{D}P_{D}Y_{D}R_{D}L_{D}W_{D}H_{D}Y_{D}P_{D}X_{D}T_{D}V_{D}N_{D}F_{D}T_{D}I_{D}F_{D}K_{D}V_{D}R_{D}M_{D}Y_{D}V_{D}GGM_{D}E_{D}H_{D}R_{D}-NH₂, wherein X is as defined above, or part thereof.

Peptide sequences defined herein are represented by one-letter symbols for amino acid residues as follows:
A (alanine)
R (arginine)
N (asparagine)
D (aspartic acid)
C (cysteine)
Q (glutamine)
E (glutamic acid)
G (glycine)
H (histidine)
I (isoleucine)
L (leucine)
K (lysine)
M (methionine)
F (phenylalanine)
P (proline)
S (serine)
T (threonine)
W (tryptophan)
Y (tyrosine)
V (valine)

Nucleic acids encoding the polypeptides of the invention are also included in the scope of the invention. Such nucleic acids may be DNA or RNA. In one embodiment, an isolated DNA molecule of the invention encodes for a peptide sequence YPYRLWHYPCTVNFTIFKVRMYVGGMEHR or a related amino acid sequence. The nucleic acid molecules of the invention may be synthetic, purified, or isolated molecules. The nucleic acid molecules can be comprised in vectors such as, but not limited to, plasmids or viruses, including expression vectors. Such techniques are well known in the art.
A particular object of this invention is a vector comprising a nucleic acid encoding a polypeptide according to the present invention. The vector may be a cloning vector or, more preferably, an expression vector, i.e., a vector comprising regulatory sequences causing expression of the polypeptide of the invention from said vector in a competent host cell.

These vectors can be used to express a polypeptide according to the invention *in vitro, ex vivo* or *in vivo.*

The vectors of this invention typically comprise a coding sequence according to the present invention "operably linked" to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

A further object of the present invention resides in a recombinant host cell comprising a recombinant gene or a vector as defined above. Suitable host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

The present invention also relates to a method for producing a recombinant host cell expressing a polypeptide according to the present invention, said method comprising (i) introducing *in vitro* or *ex vivo* into a competent host cell a recombinant nucleic acid or a vector as described above, (ii) culturing *in vitro* or *ex vivo* the recombinant host cells obtained and (iii), optionally, selecting the cells which express the polypeptide of the invention.

Such recombinant host cells can be used for the production of polypeptides, as well as for screening of active molecules, as described below.

These cells can be maintained in suitable culture media, such as DMEM, RPMI, HAM, etc., in any appropriate culture device (plate, flask, dish, tube, pouch, etc.).

The polypeptides of the invention can be synthesized by any technique commonly known in the art or may be encoded by nucleic acid as defined above, such as RNA or DNA, delivered to the host, as described above. Alternatively, they can be synthesized on a peptide synthesizer using standard solid-phase synthesis techniques and using standard Fmoc/t-butyl chemistry, as described in Merrifield R.B., Science 232, 341-347, 1986.

The compounds of the present invention may include salts thereof. When the compounds according to the invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

In another embodiment, this invention provides a pharmaceutical composition comprising at least one polypeptide as defined above or at least one nucleic acid encoding the same, as defined above. In particular, the pharmaceutical composition comprises a polypeptide which has the following sequence: YPYRLWHYPα-aminobutyrateTVNFTIFKVRMYVGGMEHR-NH₂.

The pharmaceutical composition generally additionally comprises a pharmaceutically acceptable vehicle or carrier. Because of their viral inhibitory activity, such pharmaceutical compositions possess utility in preventing or treating retroviral infection, such as HCV infection, and related disorders. In particular, such pharmaceutical compositions possess utility in inhibiting cell-to-cell transmission of retrovirus, in particular HCV, as well as virus-to-cell infection.

In yet another embodiment, the present invention discloses methods for preparing pharmaceutical compositions comprising the inventive compounds as an active ingredient.

The present invention provides polypeptides that are useful as inhibitors of viral infection, in particular as inhibitors of HCV infection. These polypeptides are more particularly able to inhibit the fusion of the virus, in particular HCV, with the target cells and more specifically with hepatocytes, as well as cell-to-cell infection.

Another embodiment of the invention discloses the use of a pharmaceutical composition defined above for the prevention or the treatment of diseases, in particular for treating diseases related or due to an infection by a virus, in particular retrovirus, including, for example, HCV. The method comprises administering a therapeutically effective amount of the inventive pharmaceutical composition to a mammal (in particular a patient) in need of such a treatment.

The present invention relates to a method for the prevention or the treatment of a disease, and in particular a disease related or due to an infection by a virus; in particular retrovirus, including HCV. More particularly, the present invention relates to a method to prevent or treat HCV infection and the like, such as the complications of HCV infection, in particular chronic hepatitis, cirrhosis, and hepatocellular carcinoma, comprising administering to a mammal (in particular a patient) in need of such treatment an effective amount of at least one polypeptide of the present invention as defined herein.

A further object of this invention is the use of an effective amount of at least one polypeptide or a nucleic acid encoding the same as defined above, for the preparation of pharmaceutical composition for the prevention or the treatment of a disease associated or due to an infection by a virus, in particular a retrovirus. More particularly, the pharmaceutical composition is intended to prevent or treat a disease associated with HCV infection.

Because of their anti-viral properties and particularly their HCV inhibitory fusion activity, the polypeptides of this invention are suitable for treating a variety of complications due to retroviral infection, in particular HCV.

In this regard, "treatment" or "treating" includes both therapeutic and prophylactic treatments. Accordingly, the compounds may be used at very early stages of a disease, or before early onset, or after significant progression.

The polypeptides of this invention are particularly suited for the treatment of HCV and complications thereof, in particular chronic hepatitis, cirrhosis, and hepatocellular carcinoma.

In the pharmaceutical compositions, uses and methods of the present invention, the active ingredients are as described above, including the defined particular embodiments.

The active ingredients will typically be administered in admixture with suitable carrier materials suitably selected with respect to the intended form and administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices.

The polypeptides or nucleic acids encoding the same may thus be formulated in various forms, including solid and liquid forms, such as tablets, capsules, gel, syrup, powder, aerosol, suppositories etc.
The compositions of this invention may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.
Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.
Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

Additionally, the compositions of the present invention may be formulated in a sustained release form to provide the rate controlled release of any one or more of the components or active ingredients to optimize the therapeutic effects, i.e. HCV inhibitory activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and pacifiers for oral solutions, suspensions and emulsions. Liquid form preparations may also include solutions for intranasal administration.
Aerosol preparations suitable for inhalation may include solutions and solids in powder form, which may be in combination with a pharmaceutically acceptable carrier such as inert gas, e.g. nitrogen.
For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.
Also included are solid form preparations which are intended to be converted, shortly before use, to liquid form preparations for either oral or parental administration. Such liquid forms include solutions, suspensions and emulsions.
The polypeptides and nucleic acids encoding the same of the invention may also be deliverable transdermally. The transdermal compositions may take the form of creams, lotions, aerosols and/or emulsions and can be included in the transdermal patch of the matrix or reservoir type as are conventional in the art for this purpose.

Preferably, the polypeptides and nucleic acids encoding the same are administered orally, intravenously or subcutaneously.

The dosages and dosage regimen in which the polypeptides of the invention and nucleic acids encoding the same are administered will vary according to the dosage form, mode of administration, the condition being treated and particulars of the patient being treated. Accordingly, optimal therapeutic concentrations will be best determined at the time and place through routine experimentation.

The polypeptides according to the invention and nucleic acids encoding the same can be used enterally or parenterally. Orally, the polypeptides according to the invention are suitably administered at the rate of 100 µg to 100 mg per day per kg of body weight. The required dose can be administered in one or more portions. For oral administration, suitable forms are, for example, tablets, gel, aerosols, pills, dragees, syrups, suspensions, emulsions, solutions, powders and granules. A preferred method of administration consists in using a suitable form containing from 1 mg to about 500 mg of active substance.

The active ingredients can also be administered parenterally in the form of solutions or suspensions for intravenous or intramuscular perfusions or injections. In that case, the compounds according to the invention are generally administered at the rate of about 10 µg to 10 mg per day per kg of body weight; a preferred method of administration consists of using solutions or suspensions containing approximately from 0.01 mg to 1 mg of active substance per ml .

The active ingredients of the invention can be used in a substantially similar manner to other known anti-viral agents, in particular antiretroviral agents. For the polypeptides of this invention, the anti-viral dose to be administered, whether a single dose, multiple dose, or a daily dose, will of course vary with the particular polypeptide employed because of the varying potency of the polypeptide, the chosen route of administration, the size of the recipient, and the nature of the patient's condition. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. Someone skilled in the art of HCV infection treatment and the like will be able to ascertain, without undue experimentation, appropriate protocols for the effective administration of the polypeptides of this present invention or nucleic acids encoding the same, such as by referring to the earlier published studies on compounds found to have anti-HCV properties or more generally antiviral properties.

Preferably, the pharmaceutical preparation is in a unit dosage form. In such form, the preparation is subdivided into suitable sized unit doses containing appropriate quantities of the active polypeptides, e.g., an effective amount to achieve the desired purpose.

The quantity of the active ingredient in a unit dose of preparation may be generally varied and adjusted from about 0.2 milligrams to about 1000 milligrams, preferably from about 0.5 to about 950 milligrams, more preferably from about 1.0 to about 500 milligrams, and typically from about 1 to 250 milligrams, according to the particular application. The actual dosage employed may be varied depending upon the patient's age, sex, weight and severity of the condition being treated. Such techniques are well known to those skilled in the art.

Generally, the human oral dosage form containing the active ingredients can be administered 1 or 2 times per day. The amount and frequency of the administration will be regulated according to the judgment of the attending clinician. A generally recommended daily dosage for oral administration may range from about 1.0 milligram to about 1,000 milligrams per day, in single or divided doses.

Conventional methods for preparing tablets are known. Such methods include dry methods such as direct compression and compression produced by compaction, or wet methods or other special procedures. Conventional methods for making other forms for administration such as, for example, capsules, suppositories and the like are also well known.

The active ingredients of the invention can be used alone or in combination with other antiviral agents. The other active ingredient can be administered in combination with a compound of the present invention either simultaneously, separately, or sequentially.
By "simultaneous", it is meant that the two or more compounds are administered at the same time, though not necessarily in the same composition. By "sequential", it is meant that the two or more compounds are administered within a time period such that the first of the two or more compounds is still active in the patient when administration of the second of the two or more compounds occurs.
Preferably, "sequential" means within the same 24 hours, preferably within the same 12 hours, such as within the same 6, 3, 1, half or quarter hour time period.

In an alternate embodiment, this invention provides a method for investigating virus, in particular retrovirus (e.g., HCV), infection and particularly the process of fusion of virus (e.g., HCV)., said methods implementing *in vitro* cultures of human hepatocytes or other target cells and polypeptides described herein.
In this respect, the present invention provides a method for screening and/or characterizing compounds that present anti(retro)viral activity, in particular anti-HCV activity, by implementing *in vitro* cultures of human peripheral blood cells described herein. In particular said methods comprise:
a) contacting a test compound with the *in vitro* cultures of peripheral blood cells in presence of virus, in particular a retrovirus (e.g. HCV or feline leukaemia virus) or active part thereof, and
b) determining the antiviral activity of the test compound in comparison with the antiviral activity of one of the polypeptides of the present invention.

In another embodiment, the invention discloses methods for inhibiting transmission of virus, in particular retrovirus, such as HCV or feline leukaemia virus, in a mammal comprising the step of administrating to said mammal any of the pharmaceutical compositions and combinations described above. If the pharmaceutical composition comprises only a polypeptide of this invention as the active component, such methods may additionally comprise the step of administering to said mammal an agent selected from an other anti(retro)viral agent, or an inhibitor of other targets in the (retro)virus life cycle. Such additional agent may be administered to the mammal prior to, concurrently with, or following the administration of the composition of the invention.
Such additional agent may be administered to the mammal prior to, concurrently with, or following the administration of the composition according to this invention.

The polypeptides set forth herein may also be used as laboratory reagents. The polypeptides of this invention may be used to treat or prevent viral contamination of materials and therefore reduce the risk of viral infection of laboratory or medical personnel or patients who come in contact with such materials. These materials include, but are not limited to, biological materials, such as blood, tissue, etc; surgical instruments and garments; laboratory instruments and garments; and blood collection apparatuses and materials.

Further aspects and advantages of this invention will be disclosed in the following examples, which should be regarded as illustrative and not limiting the scope of this application.

### LEGEND OF FIGURES

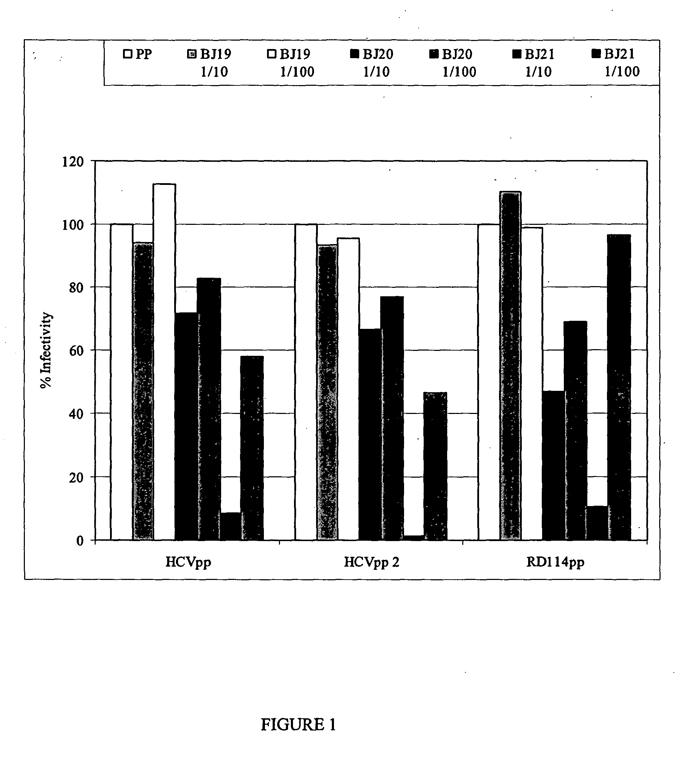

FIGURE 1: Infectivity of Huh 7 cells by HCVpp and RD114pp in the presence or in the absence (100 % infectivity) of a polypeptide (noted BJ21) according to the invention. Other peptides, noted BJ19 et BJ20, derived from E2 and CD81 respectively, are shown for the sake of comparison.

### EXAMPLES

### Methods

Peptide synthesis - Chemical synthesis of the peptides was performed by the solid phase technique using Fmoc/t-butyl chemistry, as described (Merrifield R.B., Science 232, 341-347, 1986). The crude peptides were purified to homogeneity (> 99%) by C18 reverse-phase high-pressure liquid chromatography. The purified peptides were characterized by amino acid analysis and mass spectrometry (matrix-assisted laser desorption ionisation time-of-flight technique) prior to testing.

### Antiviral activity

Evaluation of antiviral activity of the polypeptide of the following sequence:
YPYRLWHYPXTVNFTIFKVRMYVGGMEHR-NH₂ (noted BJ21 in figure 1) Said peptide was tested at 10⁻⁴ and 10⁻⁵ M (indicated in figure 1 as 1/10 and 1/100 dilutions) on HCVpp genotype 1a (in duplicate) and as control on RD114pp, with Huh 7 as target cells. HCVpp genotype 1a and RD114pp (pp stands for pseudo particles) are as defined in P.-Y. Lozach et al., (2004) J. Biol. Chem. 279, 32035-32045, and B. Bartosch et al., (2003) J. Exp. Med. 197, 633-642. Infection in the absence of peptides was standardized to 100%. Virus and peptides were added to target Huh7 cells at the same time, without preincubation. Other peptides, noted BJ19 et BJ20 in figure 1, respectively derived from E2 and CD81 peptides sequences, were tested for the sake of comparison.
BJ 19, derived from E2 primary sequence, presents the following sequence:
NWTRGERCDLEDRDRSELSP-NH2 (SEQ ID N°3)
BJ 20, derived from CD81 primary sequence, presents the following sequence:
KNNLCPSGSNIISNLFKEDCHQKIDDLFSGK-NH2 (SEQ ID N°4)

### Results

Results given by Figure 1 show the inhibitory effect on viral infection of a polypeptide of the invention in comparison with other polypeptides.

## Claims

1. Use of an effective amount of a compound selected from a polypeptide comprising the amino acid sequence (I) : YPYRLWHYPXTVNFTIFKVRMYVGGMEHR (I) (SEQ ID No. 1), wherein X is C or an amino acid derivative thereof devoid of SH group, such as α-aminobutyrate, or an equivalent amino acid residue; a fragment thereof; a variant thereof; a polypeptide-mimicking molecule; and a nucleic acid encoding the same; for the preparation of pharmaceutical composition intended for the prevention or treatment of a disease associated with an infection by a virus.

2. Use according to claim 1, wherein the polypeptide comprises the following amino acid sequence: GPWLTPRXIVDYPYRLWHYPXTVNFTIFKVRMYVGGMEHR (II) (SEQ ID No. 2).

3. Use according to claim 1 or 2, wherein X is C, A, S, Y, T or an amino acid derivative thereof devoid of SH group.

4. Use according to any one of claims 1 to 3, wherein the virus is a retrovirus.

5. Use according to any one of claims 1 to 3, wherein the virus is HCV.

6. Use according to any one of claims 1 to 3, wherein the virus is HCV or feline leukemia virus.

7. Use according to any one of the preceding claims, wherein the polypeptides are from three amino acid residues to fifty amino acid residues in length, more preferably from five amino acid residues to twenty, thirty or forty amino acid residues in length.

8. Use according to any one of the preceding claims, wherein the polypeptide presents either carboxylic (e.g., -COOH) or carboxyl-aminated (e.g., -CONH₂) C-terminal extremity.

9. Use according to any one of the preceding claims, wherein the polypeptide presents a lipid moiety, covalently or non covalently attached, at the N- or C- terminal extremity thereof.

10. Use according to any one of the preceding claims, of a molecule comprising two or more covalently or non covalently attached polypeptides of amino acid sequence (I) or (II).

11. Use according to any one of the preceding claims, wherein the polypeptide comprises at least one D-amino acid.

12. Use according to any one of the preceding claims, wherein the polypeptide sequence is YPYRLWHYPα-aminobutyrateTVNFTIFKVRMYVGGMEHR.

13. Use according to any one of the preceding claims, wherein the nucleic acid encoding a polypeptide as defined in claim 1 is a DNA molecule.

14. An isolated polypeptide comprising the amino acid sequence (I) :
YPYRLWHYPXTVNFTIFKVRMYVGGMEHR (I) (SEQ ID No. 1), wherein X is C or an amino acid derivative thereof devoid of SH group, such as α-aminobutyrate, or an equivalent amino acid residue; a fragment thereof; a variant thereof; or a polypeptide-mimicking molecule, wherein said polypeptide is from three amino acid residues to fifty amino acid residues in length, more preferably from five amino acid residues to twenty, thirty or forty in length.

15. A polypeptide according to claim 14, wherein said polypeptide comprises the following amino acid sequence (II) :
GPWLTPRXIVDYPYRLWHYPXTVNFTIFKVRMYVGGMEHR (SEQ ID No. 2), with X is as defined in claim 14.

16. A polypeptide according to claim 14 or 15, wherein X is C, A, S, Y, T or an amino acid derivative thereof devoid of SH group, such as α-aminobutyrate.

17. A polypeptide according to any one of claims 14 to 16, wherein said polypeptide presents a carboxyl-aminated (e.g., -CONH₂) C-terminal extremity.

18. A polypeptide according to any one of claims 14 to 17, wherein said polypeptide is selected in the group consisting of :
YPYRLWHYPα-aminobutyrateTVNFTIFKVRMYVGGMEHR, YPYRLWHYPα-aminobutyrateTVNFTIFKVRMYVGGMEHR-NH₂, GPWLTPRα-aminobutyrateIVDYPYRLWHYPα-aminobutyrateTVNFTIFKVRMYVGGMEHR, GPWLTPRα-aminobutyrateIVDYPYRLWHYPα-aminobutyrateTVNFTIFKVRMYVGGMEHR-NH₂, YPα-aminobutyrateTVNFTIFKVRMYVGGMEHR, and YPα-aminobutyrateTVNFTIFKVRMYVGGMEHR-NH₂.

19. An isolated polypeptide according to one of claims 14-18, wherein it comprises the following sequence:
YPYRLWHYPα-aminobutyrateTVNFTIFKVRMYVGGMEHR.

20. A polypeptide according to claim 19, which is YPYRLWHYPα-aminobutyrateTVNFTIFKVRMYVGGMEHR-NH₂.

21. A pharmaceutical composition comprising at least one compound as defined in any one of claims 14-20 and a pharmaceutically acceptable vehicle or support.

22. A pharmaceutical composition according to claim 21, said composition further comprising at least one other antiviral agent.

23. A pharmaceutical composition according to claim 21 or 22, useful for treating a disease related to an infection by a virus, in particular a retrovirus, including, for example, HCV and feline leukaemia virus.

24. A pharmaceutical composition according to any one of claims 21 to 23, useful for treating HCV infection.

25. A pharmaceutical composition according to any one of claims 21 to 24, useful for treating HCV infection and complications thereof, in particular hepatitis, cirrhosis and hepatocellular carcinoma.

26. A method for screening and/or characterizing compounds that present antiretroviral activity, said method comprising:
c) contacting a test compound *in vitro* with a culture of peripheral blood cells or hepatocytes in the presence of a retrovirus or an active part thereof, and
d) determining the antiretroviral activity of the test compound in comparison with the antiretroviral activity of one of the polypeptides as defined in any one of claims 1-20.

27. Use of at least one polypeptide as defined in any of claims 1-20 as an agent to treat or prevent viral contamination of materials.


SEQUENCE LISTING

<110>  GENOSCIENCE

<120>  COMPOUNDS FOR TREATING HCV INFECTION BY INHIBITION OF THE FUSION OF
THE VIRUS

<130>  B0333EP

<160>  4

<170>  PatentIn version 3.1

<210>  1
<211>  29
<212>  PRT
<213>  artificial sequence

<220>
<223>  antiviral agent
<220>
<221>  MISC_FEATURE
<222>  (10)..(10)
<223>  Cystein or derivative thereof devoid of SH

<400>  1
Tyr Pro Tyr Arg Leu Trp His Tyr Pro Xaa Thr Val Asn Phe Thr Ile
1               5                   10                  15

Phe Lys Val Arg Met Tyr Val Gly Gly Met Glu His Arg
            20                  25

<210>  2
<211>  40
<212>  PRT
<213>  artificial sequence

<220>

<223>  antiviral agent

<220>

<221>  MISC_FEATURE

<222>  (8)..(8)

<223>  Cystein or a derivative thereof devoid of SH


<220>

<221>  MISC_FEATURE

<222>  (21)..(21)

<223>  Cystein or a derivative thereof devoid of SH


<400>  2

Gly Pro Trp Leu Thr Pro Arg Xaa Ile Val Asp Tyr Pro Tyr Arg Leu
1               5                   10                  15

Trp His Tyr Pro Xaa Thr Val Asn Phe Thr Ile Phe Lys Val Arg Met
            20                  25                  30

Tyr Val Gly Gly Met Glu His Arg
        35                  40

<210>  3

<211>  20

<212>  PRT

<213>  artificial sequence


<220>

<223>  antiviral agent

<220>

<221>  MOD_RES

<222>  (20)..(20)

<223>  C terminal AMIDATION


<400>  3

Asn Trp Thr Arg Gly Glu Arg Cys Asp Leu Glu Asp Arg Asp Arg Ser
1               5                   10                  15

Glu Leu Ser Pro
            20

<210>  4

<211>  31

<212>  PRT

<213>  artificial sequence

<220>

<223>  antiviral agent

<220>

<221>  MOD_RES

<222>  (31)..(31)

<223>  C-terminal amydation

<400>  4

Lys Asn Asn Leu Cys Pro Ser Gly Ser Asn Ile Ile Ser Asn Leu Phe
1               5                   10                  15

Lys Glu Asp Cys His Gln Lys Ile Asp Asp Leu Phe Ser Gly Lys
            20                  25                  30